# EUROPEAN PATENT SPECIFICATION

(11) **EP 1 515 717 B1**
(45) Date of publication and mention of the grant of the patent: **01.10.2008**
(21) Application number: 03740234.4
(22) Date of filing: 12.06.2003
(51) Int. Cl.: A61K 31/405, C07D 209/24

(54) **CALCIUM SALTS OF INDOLE DERIVED STATINS**
CALCIUMSALZE VON STATINEN AUS INDOL
SELS DE CALCIUM DE STATINES DERIVEES D'INDOLE

(30) Priority: 13.06.2002 US 388318 P
(43) Date of publication of application: 23.03.2005
(73) Proprietor: Novartis AG, 4056 Basel (CH); Novartis Pharma GmbH, 1230 Wien (AT)
(72) Inventor: CHEN, Guang-Pei, Livingston, NJ 07039 (US); KAPA, Prasad, Koteswara, Parsippany, NJ 07054 (US); SUTTON, Paul, Allen, Parsippany, NJ 07054 (US)
(74) Representative: Morris, Clive Sydney
(86) International application number: PCT/EP2003/006195
(87) International publication number: WO 2003/105837

(56) References cited:
- EP-A- 0 547 000
- WO-A-02/36563
- US-A- 5 354 772

## Description

Fluvastatin, (3R*,5S*)-(E)-7-[3-(4-fluorophenyl)-1-(1-methyl-ethyl)-1H-indol-2-yl]-3,5-dihydroxy-6-heptenoic acid of the formula 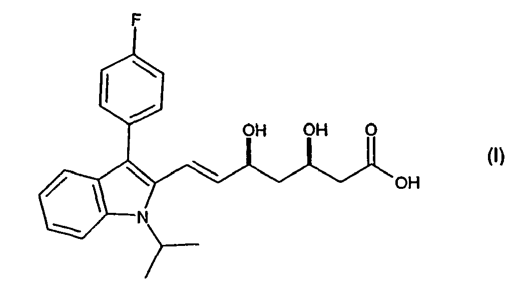 or pharmaceutically acceptable salts thereof, are inhibitors of 3-hydroxy-3-methyl-glutaryl coenzyme A (HMG-CoA) reductase. Thus, Fluvastatin, in any of its forms, may be employed for lowering of blood cholesterol levels and, therefore, for the treatment of conditions such as hypercholesteremia, hyperlipoproteinemia, dyslipidemia and atherosclerosis.

Both the racemate as well as the single enantiomers of Fluvastatin, and the sodium salts thereof, also known as Fluvastatin sodium, are disclosed in U.S. Patent No. 5,354,772. The publication by Tempkin et. al. (Tetrahedron 1997, vol. 53, 10659-10670) describes an asymmetric synthesis of the biologically most potent (3R, 5S) isomer. U.S. Patent No. 4,739,073 discloses isolation of Fluvastatin as its sodium salt by lyophilization. However, as disclosed in EP 547 000, Fluvastatin sodium is an amorphous solid and extremely susceptible to degradation at a pH about 8 or below. The suggested solution is to provide pharmaceutical compositions comprising the drug substance and an alkaline medium which is capable of sustaining the aqueous solution or dispersion of the pharmaceutical composition at a pH of at least 8. In addition to the pH sensitivity, heat and light sensitivity, as well as hygroscopicity of Fluvastatin sodium impose particular requirements on the manufacture and storage of pharmaceutical compositions comprising Fluvastatin sodium.

It is known in the art that higher crystallinity and lower hygroscopicity will lead to improved chemical stability and longer shelf life of chemical compounds. Consequently, there is a need for new forms of Fluvastatin having improved chemical stability, making possible the preparation of pharmaceutical formulations of Fluvastatin with less need for stabilizing agents and with prolonged shelf life, and with the possibility of being provided in less sophisticated packages.

In one aspect, the present invention provides calcium salts of the formula 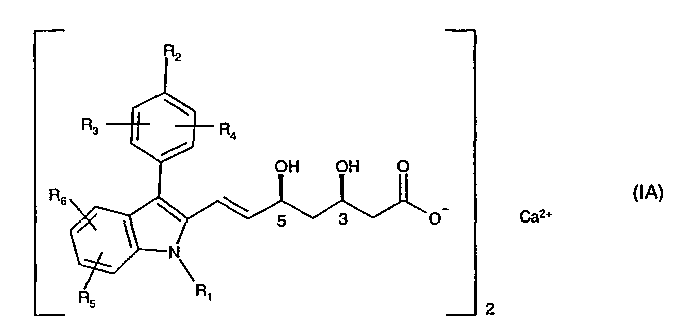 wherein R₁ is alkyl, cycloalkyl or aralkyl; R₂, R₃ and R₄ are independently hydrogen, halogen or alkyl; R₅ and R₆ are independently hydrogen, halogen, alkyl, cycloalkyl, aralkyl, alkoxy or aralkoxy; and the hydroxyl group at the 3-position is in the R-configuration and at the 5-position in the S-configuration; or an enantiomer thereof; or a hydrate thereof.

In a preferred embodiment of the present invention the calcium salt is a compound of formula IA wherein R₁ is isopropyl; R₂ is fluorine and R₃, R₄, R₅ and R₆ are hydrogen; designated herein as Fluvastatin calcium.

In another aspect, the present invention relates to a crystalline form of Fluvastatin, more specifically, a crystalline Fluvastatin calcium, and to methods for the preparation of such form of Fluvastatin.

In a further aspect, the present invention provides pharmaceutical compositions comprising crystals of Fluvastatin calcium as obtainable by the methods of the present invention, and pharmaceutically acceptable excipients, diluents or carriers thereof.

Other aspects of the present invention will become apparent to those skilled in the art from the following description, appended claims and accompanying drawings.

The present invention relates to calcium salts of indole derived statins, more specifically, to optically active calcium salts of Fluvastatin, designated herein as Fluvastatin calcium, and to methods for the preparation of such forms of Fluvastatin, and to pharmaceutical compositions comprising the crystalline forms of Fluvastatin.

Listed below are definitions of various terms used to describe the compounds of the present invention. These definitions apply to the terms as they are used throughout the specification unless they are otherwise limited in specific instances either individually or as part of a larger group.

The term "alkyl" refers to straight or branched chain hydrocarbon groups having 1 to 20 carbon atoms, preferably 1 to 7 carbon atoms. Exemplary unsubstituted alkyl groups include methyl, ethyl, propyl, isopropyl, n-butyl, t-butyl, isobutyl, pentyl, hexyl, isohexyl, heptyl, 4,4-dimethylpentyl, octyl and the like.

The term "lower alkyl" refers to those alkyl groups as described above having 1 to 7, preferably 1 to 4 carbon atoms.

The term "halogen" or "halo" refers to fluorine, chlorine, bromine and iodine.

The term "cycloalkyl" refers to optionally substituted monocyclic, bicyclic or tricyclic hydrocarbon groups of 3 to 12 carbon atoms, each of which may be substituted by one or more substituents such as alkyl, halo, oxo, hydroxy, alkoxy, alkylthio, nitro, cyano, trifluoromethyl and the like.

Exemplary monocyclic hydrocarbon groups include but are not limited to cyclopropyl, cyclobutyl, cyclopentyl, cyclopentenyl, cyclohexyl and cyclohexenyl and the like.

Exemplary bicyclic hydrocarbon groups include bornyl, indyl, hexahydroindyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl and the like.

Exemplary tricyclic hydrocarbon groups include adamantyl and the like.

The term "alkoxy" refers to alkyl-O-.

The term "alkylthio" refers to alkyl-S-.

The term "aralkyl" refers to an aryl group bonded directly through an alkyl group, such as benzyl and phenethyl.

The term "aralkoxy" refers to an aryl group bonded directly through an alkoxy group.

The term "aryl" refers to monocyclic or bicyclic aromatic hydrocarbon groups having 6 to 12 carbon atoms in the ring portion, such as phenyl, naphthyl, tetrahydronaphthyl, biphenyl and diphenyl groups.

In one aspect, the present invention provides calcium salts of the formula 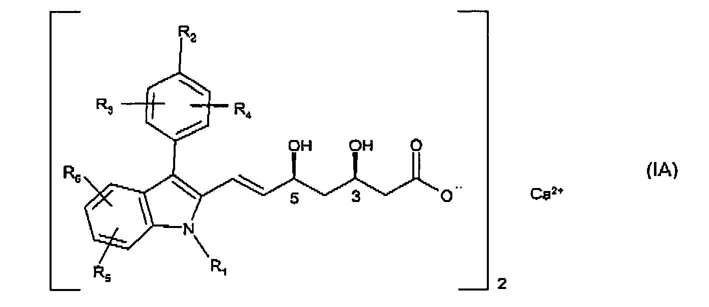 wherein R₁ is alkyl, cycloalkyl or aralkyl; R₂, R₃ and R₄ are independently hydrogen, halogen or alkyl; R₅ and R₆ are independently hydrogen, halogen, alkyl, cycloalkyl, aralkyl, alkoxy or aralkoxy; and the hydroxyl group at the 3-position is in the R-configuration and at the 5-position in the S-configuration; or an enantiomer thereof; or a hydrate thereof; which are prepared by first hydrolyzing a compound of the formula 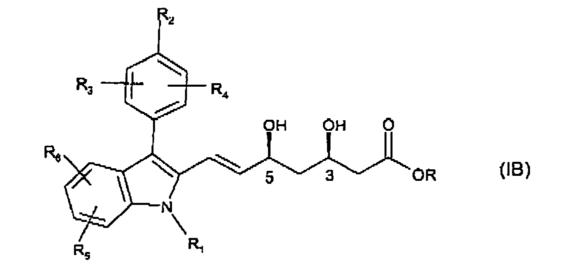 wherein R₁, R₂, R₃, R₄, R₅ and R₆ have meanings as defined for formula IA; R represents lower alkyl; and the hydroxyl group at the 3-position is in the R-configuration and at the 5-position in the S-configuration; or an enantiomer thereof; in the presence of an aqueous base, preferably an alkali metal hydroxide, to afford alkali metal salts of the formula 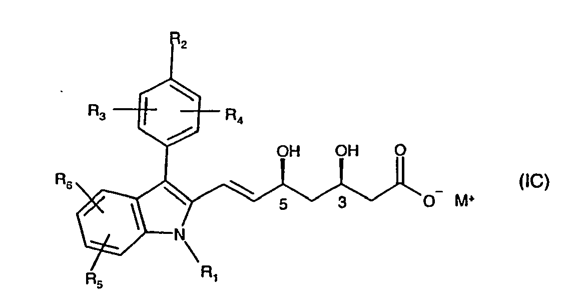 wherein M represents sodium, lithium or potassium, preferably M is sodium.

Alkali metal salts of formula IC wherein R₁, R₂, R₃, R₄, R₅, R₆ and M have meanings as defined herein above; and the hydroxyl group at the 3-position is in the R-configuration and at the 5-position in the S-configuration; or an enantiomer thereof; or a hydrate thereof; are then treated with a suitable calcium compound to afford the corresponding calcium salt of formula IA. Suitable calcium compounds include, but are not limited to, calcium chloride, calcium oxide and calcium hydroxide. Preferably, the calcium compound is calcium chloride.

In a preferred embodiment of the present invention said calcium salt is a compound of formula IA wherein R₁ is isopropyl; R₂ is fluorine; and R₃, R₄, R₅ and R₆ are hydrogen; designated herein as Fluvastatin calcium.

In a further aspect, the present invention provides a crystalline form of Fluvastatin, more specifically, a crystalline form of Fluvastatin calcium, and methods for the preparation of such form of Fluvastatin.

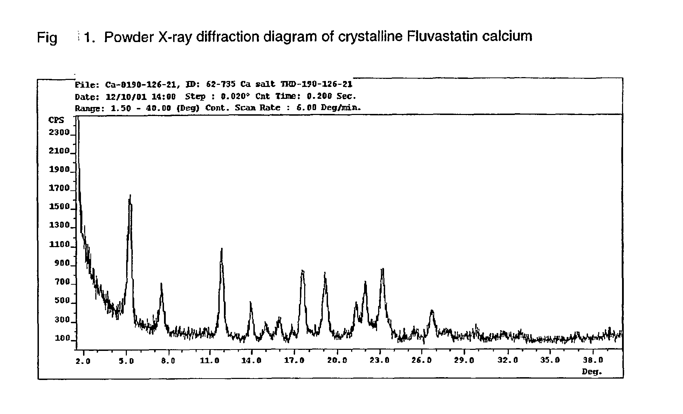

In particular, the present invention is directed to a crystalline Fluvastatin calcium which is characterized by the following physical properties: the powder X-ray diffraction diagram shows maxima at 2θ values of 5.3, 11.8, 13.9, 17.5, 19.1, 22.0 and 23.1 (Figure 1); and the melting point has been found to be about 220°C.

The melting point has been measured by Differential Scanning Calorimetry (DSC) method using Seiko DSC 22C system, and the powder X-ray diffraction diagram has been recorded between 1.5° and 40° (2 theta, i.e., 2θ) with CuK∝ radiation using a Scintag XDS diffraction system.

A discussion of the theory of powder X-ray diffraction patterns can be found in "X-ray diffraction procedures" by Klug and Alexander, J. Wiley, New York (1974), and as pointed out, e.g., by Li et. al. in J. Pharm. Sci., pages 337-346 (1999), enantiomers have same solid state properties, like melting points and X-ray data.

The crystalline Fluvastatin calcium defined herein above may be prepared as described generally herein for calcium salts of formula IA, i.e., by first hydrolyzing a compound of the formula 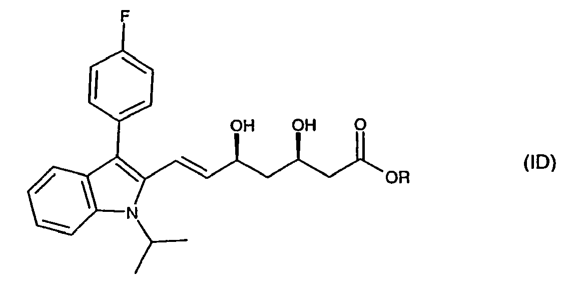 wherein R represents lower alkyl, preferably methyl, ethyl or t-butyl; and the hydroxyl group at the 3-position is in the R-configuration and at the 5-position in the S-configuration; or an enantiomer thereof; in the presence of an aqueous base, preferably an alkali metal hydroxide, to afford alkali metal salts of the formula 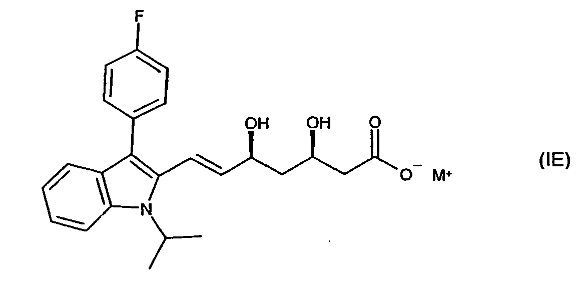 wherein M represents sodium, lithium or potassium, preferably sodium.

The hydrolysis step above may be carried out in the presence of an organic solvent such as a lower alcohol, preferably ethanol, and the aqueous base used to accomplish the hydrolysis is preferably selected from the group consisting of potassium hydroxide, lithium hydroxide and sodium hydroxide. More preferably, the base is sodium hydroxide. The hydrolysis is preferably conducted at a temperature ranging from about 0°C to about 45°C, preferably from about 20°C to about 35°C.

Alkali metal salts of formula IE may be isolated by lyophilization as described for Fluvastatin sodium in U.S. Patent No. 4,739,073, and then converted to the crystalline Fluvastatin calcium defined herein above, or preferably, the alkali metal salts of formula IE may be converted to the crystalline Fluvastatin calcium without isolation as described herein in the illustrative Examples.

Accordingly, the crystalline Fluvastatin calcium may be obtained by reacting an aqueous solution of an alkali metal salt of formula IE, preferably a sodium salt of formula IE, with an aqueous solution of a suitable calcium compound at an ambient temperature, preferably at room temperature. Suitable calcium compounds include, but are not limited to, calcium chloride, calcium oxide and calcium hydroxide. Preferably, the calcium compound is calcium chloride.

The resulting precipitated crystals of Fluvastatin calcium, or a hydrate thereof, may be isolated using conventional methods such as vacuum filtration or centrifugation, preferably vacuum filtration. The crystals may be washed, preferably with water, and then dried under atmospheric or reduced pressure, preferably under reduced pressure ranging from about 20 to about 0.1 mmHg, at a temperature ranging from room temperature to about 50°C for a period ranging from about 24 to about 72 h.

The processes described herein above may be conducted under inert atmosphere, preferably under nitrogen atmosphere.

The above disclosed methods for the preparation of Fluvastatin calcium give Fluvastatin in a stable crystalline form which is preferable to Fluvastatin sodium produced by lyophilization as an amorphous solid.

In a further aspect, the present invention provides pharmaceutical compositions comprising crystals of Fluvastatin calcium, e.g. as obtainable by the above methods, and pharmaceutically acceptable excipients, diluents or carriers thereof, especially for the prevention and/or treatment e.g. of hypercholesterolemia, hyperlipoproteinemia, dyslipidemia and atherosclerosis in mammals.

The present invention also relates to the use of Fluvastatin calcium in the manufacture of a pharmaceutical composition comprising mixing an effective amount of crystals of Fluvastatin calcium as obtainable by the above methods, and pharmaceutically acceptable excipients, diluents or carriers thereof, especially for the prevention and/or treatment e.g. of hypercholesterolemia, hyperlipoproteinemia, dyslipidemia and atherosclerosis in mammals.

Fluvastatin, in any of its forms, may be employed therapeutically for lowering of blood cholesterol levels, and therefore, as a hypercholesterolemic, hyperlipoproteinemic, dyslipidemic and antiatherosclerotic agent. Accordingly, described is a method for the prevention and/or treatment e.g. of hypercholesterolemia, hyperlipoproteinemia, dyslipidemia and atherosclerosis in mammals, which method comprises administering a therapeutically effective amount of crystals of Fluvastatin calcium as obtainable by the methods of the present invention.

The invention further relates to a combination, such as a pharmaceutical composition, comprising a salt of formula (I A) and at least one therapeutic agent selected from the group consisting of
(i) an AT₁-receptor antagonist or a pharmaceutically acceptable salt thereof,
(ii) a renin inhibitor or a pharmaceutically acceptable salt thereof,
(iii) an angiotensin converting enzyme (ACE) inhibitor or a pharmaceutically acceptable salt thereof,
(iv) an Calcium channel blocker or a pharmaceutically acceptable salt thereof,
(v) an aldosterone synthase inhibitor or a pharmaceutically acceptable salt thereof,
(vi) an aldosterone antagonist or a pharmaceutically acceptable salt thereof,
(vii) an dual angiotensin converting enzyme/neutral endopetidase (ACE/NEP) inhibitor or a pharmaceutically acceptable salt thereof,
(viii) an endothelin antagonist or a pharmaceutically acceptable salt thereof, and
(ix) a diuretic or a pharmaceutically acceptable salt thereof.

The term "at least one therapeutic agent" shall mean that in addition to the compound of formula (I) one or more, for example two, furthermore three, active ingredients as specified according to the present invention can be combined.
AT₁-receptor antagonists (also called angiotensin II receptor antagonists) are understood to be those active ingredients that bind to the AT₁-receptor subtype of angiotensin II receptor but do not result in activation of the receptor. As a consequence of the inhibition of the AT₁ receptor, these antagonists can, for example, be employed as antihypertensives or for treating congestive heart failure.

The class of AT₁ receptor antagonists comprises compounds having differing structural features, essentially preferred are the non-peptidic ones. For example, mention may be made of the compounds that are selected from the group consisting of valsartan (cf. EP 443983), losartan (cf. EP25331 0), candesartan (cf. 459136), eprosartan (cf. EP 403159), irbesartan (cf. EP454511), olmesartan (cf. EP 503785), tasosartan (cf. EP539086), telmisartan (cf. EP 522314), the compound with the designation E-1477 of the following formula 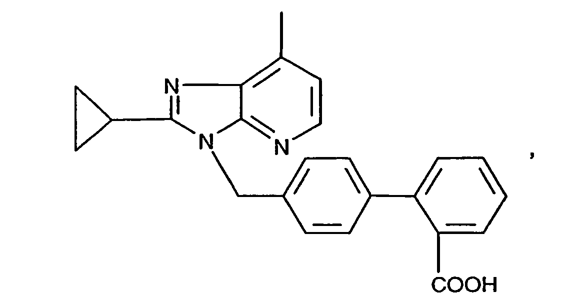 the compound with the designation SC-52458 of the following formula 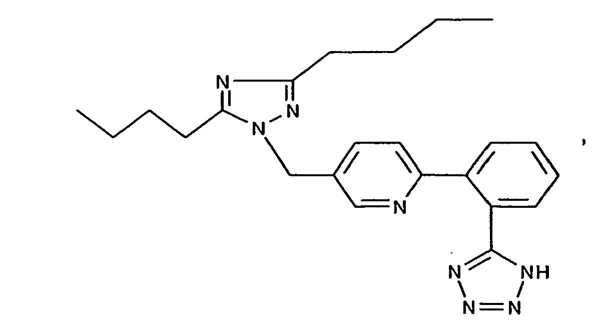 and the compound with the designation the compound ZD-8731 of the following formula 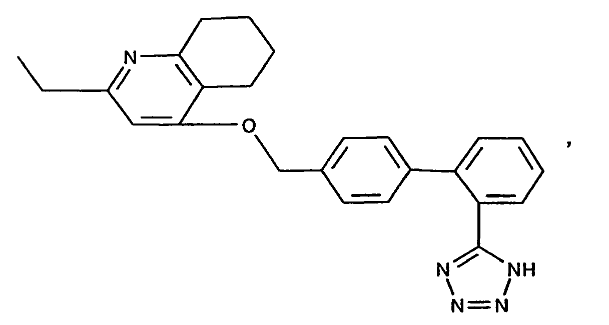 or, in each case, a pharmaceutically acceptable salt thereof.

Preferred AT₁-receptor antagonist are those agents that have been marketed, most preferred is valsartan or a pharmaceutically acceptable salt thereof.

A preferred renin is aliskiren, chemically defined as 2(S),4(S),5(S),7(S)-N-(3-amino-2,2-dimethyl-3-oxopropyl)-2,7-di(1-methylethyl)-4-hydroxy-5-amino-8-[4-methoxy-3-(3-methoxy-propoxy)phenyl]-octanamide, is specifically disclosed in EP 678503 A. Especially preferred is the hemi-fumarate salt thereof.

The interruption of the enzymatic degradation of angiotensin I to angiotensin II with so-called ACE-inhibitors (also called angiotensin converting enzyme inhibitors) is a successful variant for the regulation of blood pressure and thus also makes available a therapeutic method for the treatment of congestive heart failure.

The class of ACE inhibitors comprises compounds having differing structural features. For example, mention may be made of the compounds which are selected from the group consisting alacepril, benazepril, benazeprilat, captopril, ceronapril, cilazapril, delapril, enalapril, enaprilat, fosinopril, imidapril, lisinopril, moveltopril, perindopril, quinapril, ramipril, spirapril, temocapril, and trandolapril, or, in each case, a pharmaceutically acceptable salt thereof.

Preferred ACE inhibitors are those agents that have been marketed, most preferred are benazepril and enalapril.

The class of CCBs essentially comprises dihydropyridines (DHPs) and non-DHPs such as diltiazem-type and verapamil-type CCBs.

A CCB useful in said combination is preferably a DHP representative selected from the group consisting of amlodipine, felodipine, ryosidine, isradipine, lacidipine, nicardipine, nifedipine, niguldipine, niludipine, nimodipine, nisoldipine, nitrendipine, and nivaldipine, and is preferably a non-DHP representative selected from the group consisting of flunarizine, prenylamine, diltiazem, fendiline, gallopamil, mibefradil, anipamil, tiapamil and verapamil, and in each case, a pharmaceutically acceptable salt thereof. All these CCBs are therapeutically used, e.g. as anti-hypertensive, anti-angina pectoris or anti-arrhythmic drugs.

Preferred CCBs comprise amlodipine, diltiazem, isradipine, nicardipine, nifedipine, nimodipine, nisoldipine, nitrendipine, and verapamil, or, e.g. dependent on the specific CCB, a pharmaceutically acceptable salt thereof. Especially preferred as DHP is amlodipine or a pharmaceutically acceptable salt, especially the besylate, thereof. An especially preferred representative of non-DHPs is verapamil or a pharmaceutically acceptable salt, especially the hydrochloride, thereof.

Aldosterone synthase inhibitor is an enzyme that converts corticosterone to aldosterone by hydroxylating cortocosterone to form 18-OH-corticosterone and 18-OH-corticosterone to aldosterone. The class of aldosterone synthase inhibitors is known to be applied for the treatment of hypertension and primary aldosteronism comprises both steroidal and non-steroidal aldosterone synthase inhibitors, the later being most preferred.

Preference is given to commercially available aldosterone synthase inhibitors or those aldosterone synthase inhibitors that have been approved by the health authorities.

The class of aldosterone synthase inhibitors comprises compounds having differing structural features. For example, mention may be made of the compounds which are selected from the group consisting of the non-steroidal aromatase inhibitors anastrozole, fadrozole (including the (+)-enantiomer thereof), as well as the steroidal aromatase inhibitor exemestane, or, in each case where applicable, a pharmaceutically acceptable salt thereof.

The most preferred non-steroidal aldosterone synthase inhibitor is the (+)-enantiomer of fadrozole (US patents 4617307 and 4889861) of formula 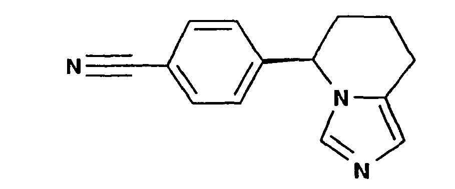 or a pharmaceutically acceptable salt thereof.

A preferred steroidal aldosterone antagonist is eplerenone (cf. EP 122232 A) of the formula 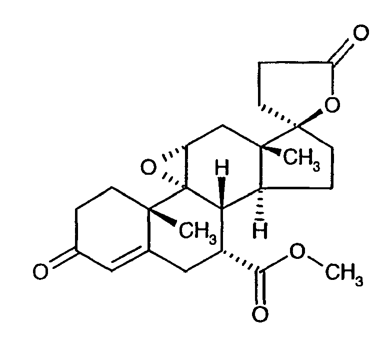 or spironolactone.

Compounds having an inhibitory effects on both angiotensin converting enzyme and neutral endopetidase, so-called dual ACE/NEP inhibitors, can be used for the treatment of cardiovascular pathologies.

A preferred dual angiotensin converting enzyme/neutral endopetidase (ACE/NEP) inhibitor is, for example, omapatrilate (cf. EP 629627), fasidotril or fasidotrilate, or Z 13752A (cf. WO 97/24342) or, if appropriable, a pharmaceutically acceptable salt thereof.

Endothelin (ET) is a highly potent vasoconstrictor peptided synthesized and released by the vascular endotleium. Endothelin exists in three isoforms (ET-1, ET-2 and ET-3). (ET shall meand any or all othe isoforms of ET). Elevated levels of ET have been reported in plasma fomr patients with e.g. essential hypertension. Endothelin receptor antagonist can be used to inhibit the vasoconstrictive effects induced by ET.

A preferred endothelin antagonist is, for example, bosentan (cf. EP 526708 A), enrasentan (cf. WO 94/25013), atrasentan (cf. WO 96/06095), especially atrasentan hydrochloride, darusentan (cf. EP 785926 A), BMS 193884 (cf. EP 702012 A), sitaxentan (cf. US 5594021), especially sitaxsentan sodium, YM 598 (cf. EP 882719 A), S 0139 (cf. WO 97/27314), J 104132 (cf. EP 714897 A or WO 97/37665), furthermore, tezosentan (cf. WO 96/19459), or in each case, a pharmaceutically acceptable salt thereof.

A diuretic is, for example, a thiazide derivative selected from the group consisting of chlorothiazide, hydrochlorothiazide, methylclothiazide, and chlorothalidon. The most preferred is hydrochlorothiazide.

The structure of the active agents identified by generic or tradenames may be taken from the actual edition of the standard compendium "The Merck Index" or from databases, e.g. LifeCycle Patents International (e.g. IMS World Publications). The corresponding content thereof is hereby incorporated by reference. Any person skilled in the art is fully enabled to identify the active agents and, based on these references, likewise enabled to manufacture and test the pharmaceutical indications and properties in standard test models, both in vitro and in vivo.

The present invention is further described by the following examples. The examples are intended to illustrate the invention and are not to be construed as being limitations thereon. If not mentioned otherwise, all evaporations are performed under reduced pressure, preferably between about 10 and 100 mmHg. The structure of final products, intermediates and starting materials is confirmed by standard analytical methods, e.g., microanalysis, melting point (mp) and spectroscopic characteristics (e.g. MS, IR, NMR).

### Example 1

### (E)-(3R,5S)-(+)-7-[3-(4-Fluoro-phenyl)-1-isopropyl-1H-indol-2-yl]-3,5-dihydroxy-hept-6-enoic acid calcium salt, Fluvastatin calcium, (+)-enantiomer

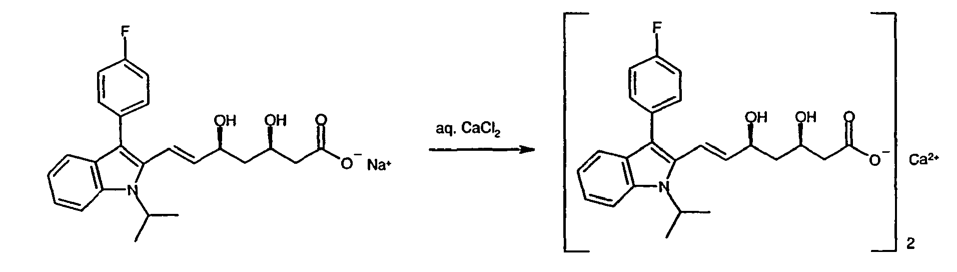

Under a nitrogen atmosphere, to a stirred solution of 15.0 g (0.0346 mol) of (E)-(3R,5S)-(+)-7-[3-(4-fluoro-phenyl)-1-isopropyl-1H-indol-2-yl]-3,5-dihydroxy-hept-6-enoic acid sodium salt in 300 mL of water is added a solution of 2.8 g (0.019 mol) of calcium chloride dihydrate in 20 mL of water at room temperature. A white precipitate starts to form immediately, and the reaction mixture gradually changes to a thick white slurry. After about 2 h, the precipitate is collected by vacuum filtration, washed twice with 50 mL of water and dried at 45°C in a vacuum oven for about 72 h to afford 14.05 g of crystalline (E)-(3R,5S)-(+)-7-[3-(4-fluorophenyl)-1-isopropyl-1H-indol-2-yl]-3,5-dihydroxy-hept-6-enoic acid calcium salt: mp 220°C; powder X-ray diffraction diagram as shown in Figure 1.

### Example 2

### (E)-(3R,5S)-(+)-7-[3-(4-Fluoro-phenyl)-1-isopropyl-1H-indol-2-yl]-3,5-dihydroxy-hept-6-enoic acid calcium salt, Fluvastatin calcium, (+)-enantiomer

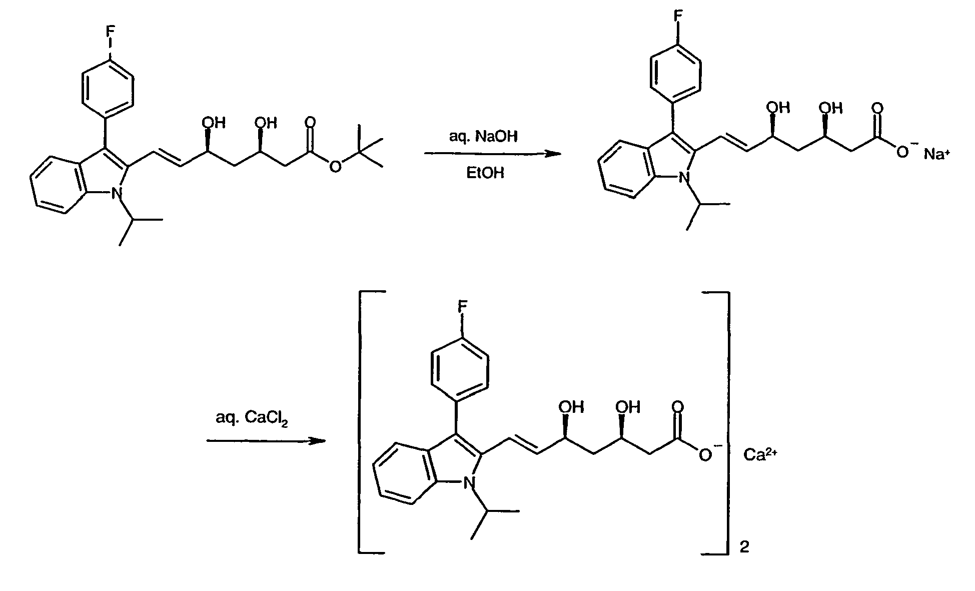

Under a nitrogen atmosphere, to a solution of 19.22 g (0.0412 mol) of (E)-(3R,5S)-(+)-7-[3-(4-fluoro-phenyl)-1-isopropyl-1H-indol-2-yl]-3,5-dihydroxy-hept-6-enoic acid 1,1-dimethylethyl ester in 95 mL of ethanol is added a solution of 1.58 g (0.0395 mol) of sodium hydroxide in 33 mL of water dropwise while maintaining the internal temperature in a range of 25±3°C. The reaction is then warmed to 33±2°C and stirred for 1.5 h further. The reaction mixture is filtered and the filtration residue is washed with 20 mL of water. To the filtrate are added 120 mL of water and 220 mL of methyl-t-butyl ether, and the mixture is stirred for 5 min. The layers are separated, and the aqueous layer is washed with 75 mL of methyl-t-butyl ether. The aqueous solution is concentrated to a volume of about 100 mL under reduced pressure (water bath -50°C), and 206 mL of water are added to form a yellow clear sodium salt solution. A solution of 3.21 g (0.0218 mol) of calcium chloride dihydrate in 40 mL of water is added dropwise while vigorously stirring. The solution immediately changes to a white slurry. Stirring is continued for 3 h further. The solid is collected by filtration, washed with 200 mL of water and dried at 45°C in a vacuum oven for 36 h to obtain 15.6 g of crystalline (E)-(3R,5S)-(+)-7-[3-(4-fluoro-phenyl)-1-isopropyl-1H-indol-2-yl]-3,5-dihydroxy-hept-6-enoic acid calcium salt as characterized by mp and powder X-ray diagram.

## Claims

1. A calcium salt of the formula 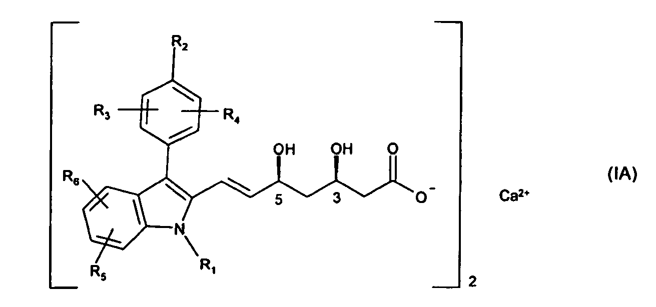 wherein R₁ is alkyl, cycloalkyl or aralkyl; R₂, R₃ and R₄ are independently hydrogen, halogen or alkyl; R₅ and R₆ are independently hydrogen, halogen, alkyl, cycloalkyl, aralkyl, alkoxy or aralkoxy; and the hydroxyl group at the 3-position is in the R-configuration and at the 5-position in the S-configuration; or an enantiomer thereof; or a hydrate thereof; obtainable by a process comprising:
(1) hydrolyzing a compound of the formula 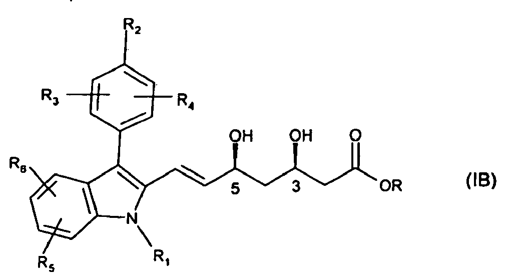 wherein R₁, R₂, R₃, R₄, R₅ and R₆ have meanings as defined for formula IA; R represents C₁-C₇ alkyl; and the hydroxyl group at the 3-position is in the R-configuration and at the 5-position in the S-configuration; or an enantiomer thereof; in the presence of an aqueous base to afford an alkali metal salt of the formula 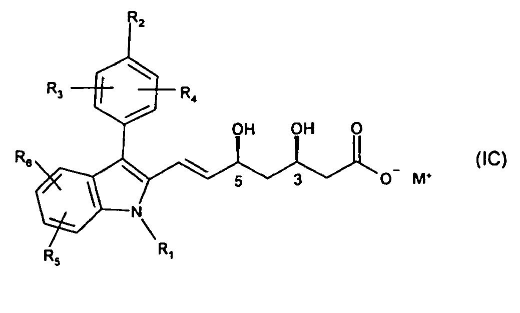 wherein M represents sodium, lithium or potassium; and
(2) treating the alkali metal salt of formula IC with a calcium compound to afford the calcium salt of formula IA.

2. A calcium salt according to claim 1, obtainable by a process wherein the aqueous base in step (1) is sodium hydroxide and M in formula IC represents sodium and wherein the calcium compound in step (2) is calcium chloride.

3. A calcium salt according to claim 1, wherein R₁ is isopropyl, R₂ is fluorine, and R₃, R₄, R₅ and R₆ are hydrogen.

4. A calcium salt of the formula 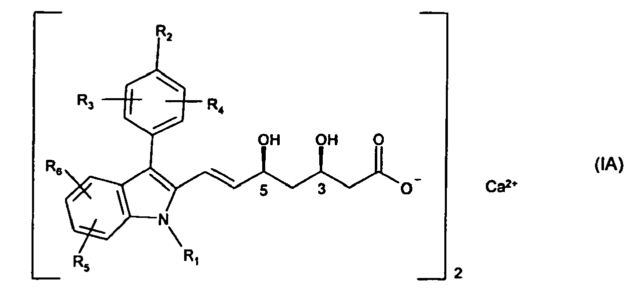 wherein R₁ is alkyl, cycloalkyl or aralkyl; R₂, R₃ and R₄ are independently hydrogen, halogen or alkyl; R₅ and R₆ are independently hydrogen, halogen, alkyl, cycloalkyl, aralkyl, alkoxy or aralkoxy; and the hydroxyl group at the 3-position is in the R-configuration and at the 5-position in the S-configuration; or an enantiomer thereof; or a hydrate thereof; obtainable by treating an alkali metal salt of the formula 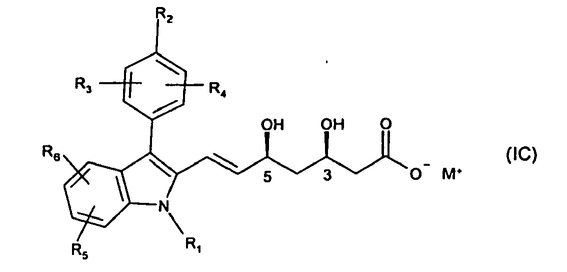 wherein R₁, R₂, R₃, R₄, R₅ and R₆ have meanings as defined for formula IA; M represents sodium, lithium or potassium; and the hydroxyl group at the 3-position is in the R-configuration and at the 5-position in the S-configuration; or an enantiomer thereof; or a hydrate thereof; with a calcium compound to afford the calcium salt of formula IA.

5. A calcium salt according to claim 4, obtainable by a process wherein M in formula IC represents sodium and the calcium compound is calcium chloride.

6. A calcium salt according to claim 4, wherein R₁ is isopropyl, R₂ is fluorine, and R₃, R₄, R₅ and R₆ are hydrogen.

7. A crystalline calcium salt according to claim 1,
wherein R₁ is isopropyl; R₂ is fluorine; R₃, R₄, R₅ and R₆ are hydrogen; and the hydroxyl group at the 3-position is in the R-configuration and at the 5-position in the S-configuration; or an enantiomer thereof; or a hydrate thereof.

8. A crystalline calcium salt according to claim 7, which has a powder X-ray diffraction pattern with maxima at 26 values of 5.3, 11.8, 13.9, 17.5, 19.1, 22.0 and 23.1 and which has a melting point of about 220°C.

9. A method for the preparation of a crystalline calcium salt according to claim 7, which method comprises:
(1) hydrolyzing a compound of the formula 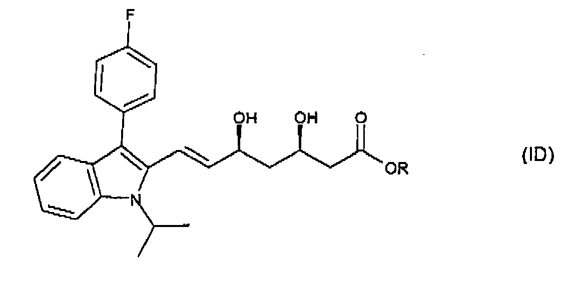 wherein R represents C₁-C₇ alkyl; and the hydroxyl group at the 3-position is in the R-configuration and at the 5-position in the S-configuration; or an enantiomer thereof; in the presence of an aqueous base to afford an alkali metal salt of the formula 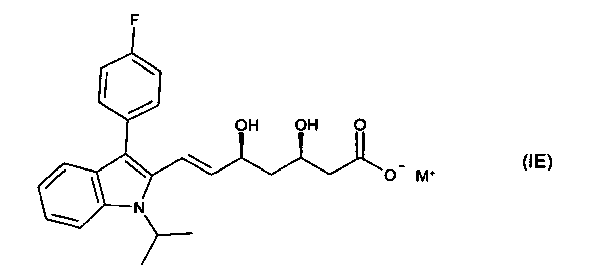 wherein M represents sodium, lithium or potassium; and
(2) treating the alkali metal salt of formula IE with a calcium compound to afford the crystalline calcium salt according to claim 7.

10. The method according to claim 9, wherein the aqueous base in step (1) is sodium hydroxide and M in formula IE represents sodium and wherein the calcium compound in step (2) is calcium chloride.

11. A method for the preparation of a crystalline calcium salt according to claim 7, which method comprises treating an alkali metal salt of the formula 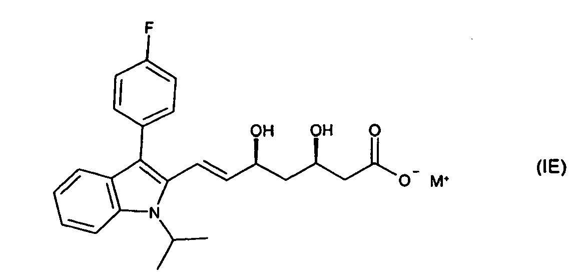 wherein M represents sodium, lithium or potassium; and the hydroxyl group at the 3-position is in the R-configuration and at the 5-position in the S-configuration; or an enantiomer thereof; or a hydrate thereof; with a calcium compound to afford the crystalline calcium salt according to claim 7.

12. The method according to claim 11, wherein M in formula IE represents sodium and the calcium compound is calcium chloride.

13. A pharmaceutical composition comprising a therapeutically effective amount of a calcium salt according to claim 7 in combination with one or more pharmaceutically acceptable carriers.

14. Use of a calcium salt according to any of claims 1 to 8, for the manufacture of a medicament for the prevention and/or treatment of hypercholesterolemia, hyperlipoproteinemia, dyslipidemia and atherosclerosis.

## Patentansprüche

1. Calciumsalz der Formel 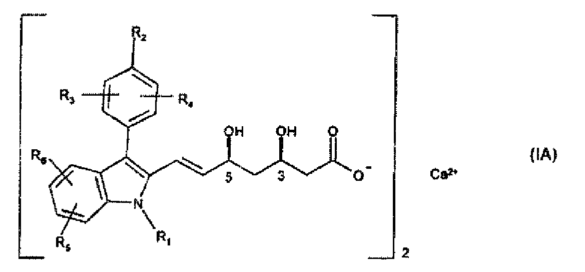 worin R₁ für Alkyl, Cycloalkyl oder Aralkyl steht, R₂, R₃ und R₄ unabhängig für Wasserstoff, Halogen oder Alkyl stehen, R₅ und R₆ unabhängig für Wasserstoff, Halogen, Alkyl, Cycloalkyl, Aralkyl, Alkoxy oder Aralkoxy stehen, und die Hydroxylgruppe an der Position 3 die R-Konfiguration und an der Position 5 die S-Konfiguration hat, oder ein Enantiomer hiervon oder ein Hydrat hiervon, erhältlich durch ein Verfahren, umfassend eine
(1) Hydrolyse der Formel 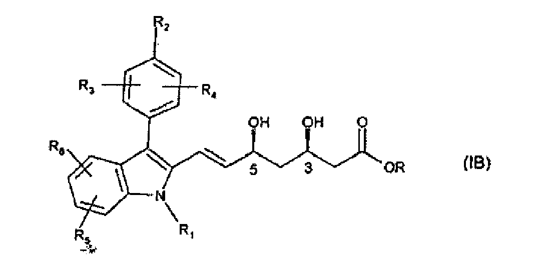 worin R₁, R₂, R₃, R₄, R₅ und R₆ die für die Formel IA definierten Bedeutungen haben, R für C₁-C₇-Alkyl steht, und die Hydroxylgruppe an der Position 3 die R-Konfiguration und an der Position 5 die S-Konfiguration hat, oder ein Enantiomer hiervon, in Gegenwart einer wässrigen Base unter Erhalt eines Alkalimetallsalzes der Formel 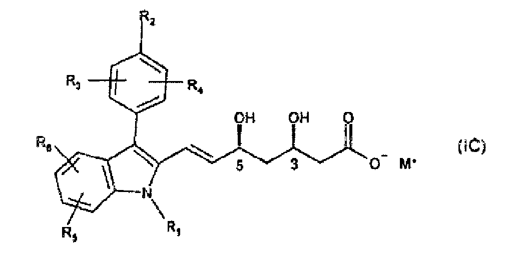 worin M für Natrium, Lithium oder Kalium steht, und
(2) Behandlung des Alkalimetallsalzes der Formel IC mit einer Calciumverbindung unter Erhalt eines Calciumsalzes der Formel IA.

2. Calciumsalz nach Anspruch 1, erhältlich durch ein Verfahren, worin die wässrige Base in der Stufe (1) Natriumhydroxid ist und M in der Formel IC für Natrium steht, und worin die Calciumverbindung in der Stufe (2) Calziumchlorid ist.

3. Calciumsalz nach Anspruch 1, worin R₁ für Isopropyl steht, R₂ für Fluor steht und R₃, R₄, R₅ und R₆ für Wasserstoff stehen.

4. Calciumsalz der Formel 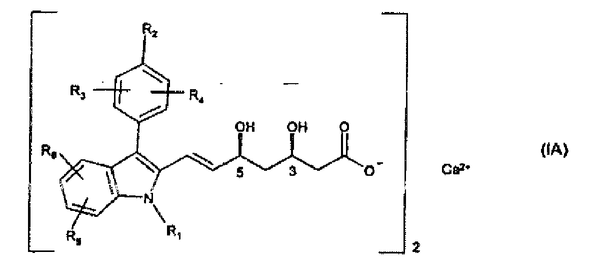 worin R₁ für Alkyl, Cycloalkyl oder Aralkyl steht, R₂, R₃ und R₄ unabhängig für Wasserstoff, Halogen oder Alkyl stehen, R₅ und R₆ unabhängig für Wasserstoff, Halogen, Alkyl, Cycloalkyl, Aralkyl, Alkoxy oder Aralkoxy stehen, und die Hydroxylgruppe an der Position 3 die R-Konfiguration und an der Position 5 die S-Konfiguration hat, oder ein Enantiomer hiervon oder ein Hydrat hiervon, erhältlich durch Behandlung eines Alkalimetallsalzes der Formel 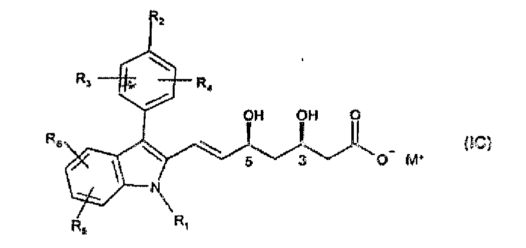 worin R₁, R₂, R₃, R₄, R₅ und R₆ die für die Formel IA definierten Bedeutungen haben, M für Natrium, Lithium oder Kalium steht, und die Hydroxylgruppe an der Position 3 die R-Konfiguration und an der Position 5 die S-Konfiguration hat, oder ein Enantiomer hiervon oder ein Hydrat hiervon, mit einer Calciumverbindung unter Erhalt des Calciumsalzes der Formel IA.

5. Calciumsalz nach Anspruch 4, erhältlich durch ein Verfahren, worin M in der Formel iC für Natrium steht und die Calciumverbindung Calciumchlorid ist.

6. Calciumsalz nach Anspruch 4, worin R₁ für Isopropyl steht, R₂ für Fluor steht und R₃, R₄, R₅ und R₆ für Wasserstoff stehen.

7. Kristallines Calciumsalz nach Anspruch 1,
worin R₁ für Isopropyl steht, R₂ für Fluor steht, R₃, R₄, R₅ und R₆ für Wasserstoff stehen, und die Hydroxylgruppe an der Position 3 die R-Konfiguration und an der Position 5 die S-Konfiguration hat, oder ein Enantiomer hiervon oder ein Hydrat hiervon.

8. Kristallines Calciumsalz nach Anspruch 7, das ein Pulver-Röntgenbeugungsspektrum mit Maxima bei 2θ bei Werten von 5,3, 11,8, 13,9, 17,5, 19,1, 22,0 und 23,1 hat und das einen Schmelzpunkt von etwa 220 °C hat.

9. Verfahren zur Herstellung eines kristallinen Calciumsalzes nach Anspruch 7, durch
(1) Hydrolyse einer Verbindung der Formel 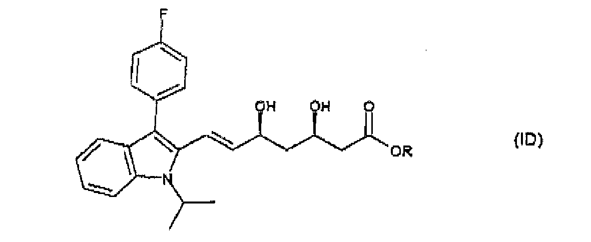 worin R für C₁-C₇-Alkyl steht und die Hydroxylgruppe an der Position 3 die R-Konfiiguration und an der Position 5 die S-Konfiguration hat, oder ein Enantiomer hiervon, in Gegenwart einer wässrigen Base unter Erhalt eines Alkalimetallsalzes der Formel 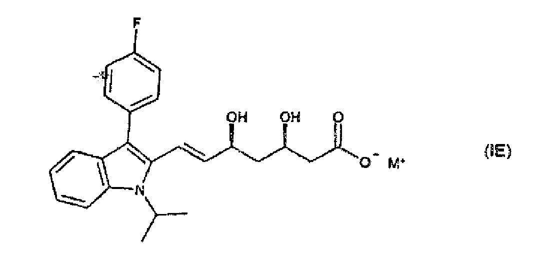 worin M für Natrium, Lithium oder Kalium steht, und
(2) Behandlung des Alkalimetallsalzes der Formel IE mit einer Calciumverbindung unter Erhalt des kristallinen Calciumsalzes nach Anspruch 7.

10. Verfahren nach Anspruch 9, worin die wässrige Base in der Stufe (1) Natriumhydroxid ist und M in der Formel IE für Natrium steht und worin die Calciumverbindung in der Stufe (2) Calciumchlorid ist.

11. Verfahren zur Herstellung eines kristallinen Calciumsalzes nach Anspruch 7, wobei dieses Verfahren umfasst eine Behandlung eines Alkalimetallsalzes der Formel 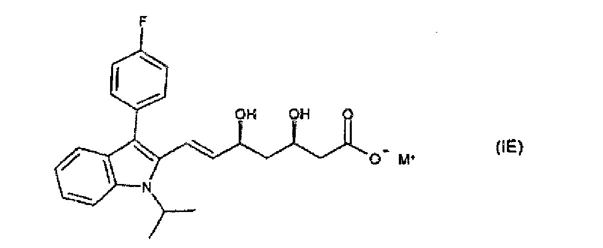 worin M für Natrium, Lithium oder Kalium steht und die Hydroxylgruppe an der Position 3 die R-Konfiguration und an der Position 5 die S-Konfiguration hat, oder ein Enantiomer hiervon oder ein Hydrat hiervon, mit einer Calciumverbindung unter Erhalt des kristallinen Calciumsalzes nach Anspruch 7.

12. Verfahren nach Anspruch 11, worin M in der Formel IE für Natrium steht und die Calciumverbindung Calciumchlorid ist.

13. Pharmazeutische Zusammensetzung, umfassend eine therapeutisch wirksame Menge eines Calciumsalzes nach Anspruch 7 in Kombination mit ein oder mehr pharmazeutisch akzeptablen Trägern.

14. Verwendung eines Calciumsalzes nach einem der Ansprüche 1 bis 8 zur Herstellung eines Arzneimittels für die Prävention und/oder Behandlung von Hypercholesterolämie, Hyperlipoproteinämie, Dyslipidämie und Atherosklerose.

## Revendications

1. Sel de calcium de formule 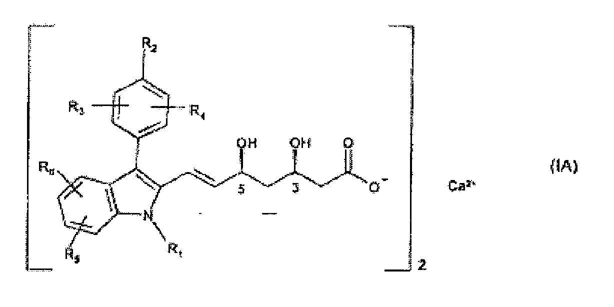 dans laquelle R₁ représente un groupe alkyle, cycloalkyle ou aralkyle ; R₂, R₃ et R₄ représentent indépendamment un hydrogène, un halogène ou un groupe alkyle ; R₅ et R₆ représentent indépendamment un hydrogène, un halogène ou un groupe alkyle, cycloalkyle, aralkyle, alcoxy ou aralcoxy ; et le groupe hydroxyle est, en position 3, dans la configuration R et, en position 5, dans la configuration S ; ou énantiomère de celui-ci ; ou hydrate de celui-ci ; pouvant être obtenu par un processus qui comprend les étapes consistant à :
(1) hydrolyser un composé de formule 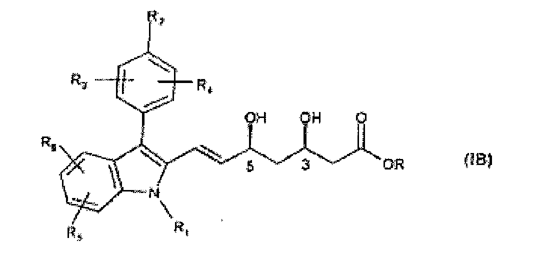 dans laquelle R₁, R₂, R₃, R₄, R₅ et R₆ ont les mêmes significations que dans la formule IA ; R représente un groupe alkyle en C₁-C₇ ; et le groupe hydroxyle est, en position 3, dans la configuration R et, en position 5, dans la configuration S ; ou un énantiomère de celui-ci ; en présence d'une base aqueuse, ce qui mène à un sel de métal alcalin de formule 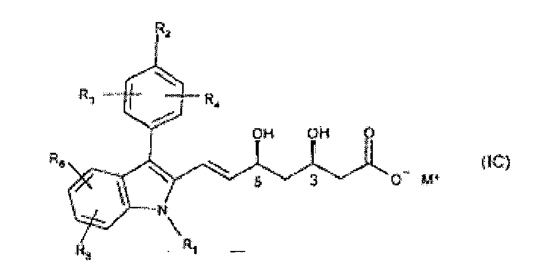 dans laquelle M représente le sodium, le lithium ou le potassium ; et
(2) traiter le sel de métal alcalin de formule IC avec un composé de calcium, ce qui mène au sel de calcium de formule IA.

2. Sel de calcium selon la revendication 1, pouvant être obtenu par un processus dans lequel la base aqueuse à l'étape (1) est l'hydroxyde de sodium et M dans la formule IC représente le sodium, et dans lequel le composé de calcium à l'étape (2) est le chlorure de calcium.

3. Sel de calcium selon la revendication 1, dans lequel R₁ représente le groupe isopropyle, R₂ représente le fluor, et R₃, R₄, R₅ et R₆ représentent l'hydrogène.

4. Sel de calcium de formule 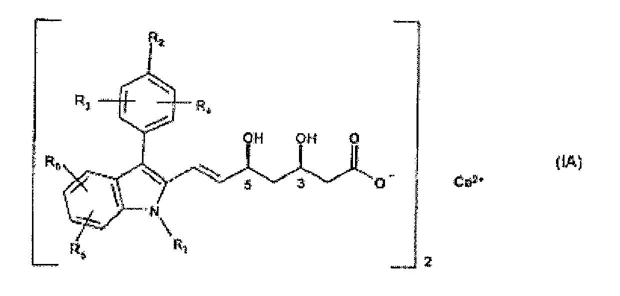 dans laquelle R₁ représente un groupe alkyle, cycloalkyle ou aralkyle ; R₂, R₃ et R₄ représentent indépendamment un hydrogène, un halogène ou un groupe alkyle ; R₅ et R₆ représentent indépendamment un hydrogène, un halogène ou un groupe alkyle, cycloalkyle, aralkyle, alcoxy ou aralcoxy ; et le groupe hydroxyle est, en position 3, dans la configuration R et, en position 5, dans la configuration S ; ou énantiomère de celui-ci ; ou hydrate de celui-ci ; pouvant être obtenu en traitant un sel de métal alcalin de formule 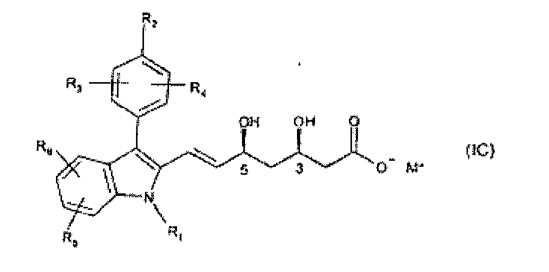 dans laquelle R₁, R₂, R₃, R₄, R₅ et R₆ ont les mêmes significations que dans la formule IA ; M représente le sodium, le lithium ou le potassium ; et le groupe hydroxyle est, en position 3, dans la configuration R et, en position 5, dans la configuration S ; ou un énantiomère de celui-ci ; ou un hydrate de celui-ci ; avec un composé de calcium, ce qui mène au sel de calcium de formule IA.

5. Sel de calcium selon la revendication 4, pouvant être obtenu par un processus dans lequel M dans la formule IC représente le sodium et le composé de calcium est le chlorure de calcium.

6. Sel de calcium selon la revendication 4, dans lequel R₁ représente le groupe isopropyle, R₂ représente le fluor, et R₃, R₄, R₅ et R₆ représentent l'hydrogène.

7. Sel de calcium cristallin selon la revendication 1, dans lequel R₁ représente le groupe isopropyle ; R₂ représente le fluor ; R₃, R₄, R₅ et R₆ représentent l'hydrogène ; et le groupe hydroxyle est, en position 3, dans la configuration R et, en position 5, dans la configuration S ; ou énantiomère de celui-ci ; ou hydrate de celui-ci.

8. Sel de calcium cristallin selon la revendication 7, qui possède un diagramme de diffraction des rayons X sur poudre présentant des maxima pour les valseurs de 2θ égales à 5,3, 11,8, 13,9, 17,5, 19,1, 22,0 et 23,1, et qui présente un point de fusion d'environ 220°C.

9. Procédé de préparation d'un sel de calcium cristallin selon la revendication 7, lequel procédé comprend les étapes consistant à :
(1) hydrolyser un composé de formule 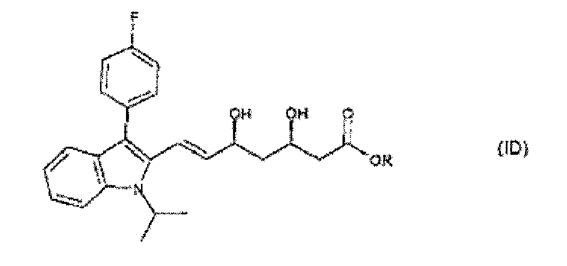 dans laquelle R représente un groupe alkyle en C₁-C₇ ; et le groupe hydroxyle est, en position 3, dans la configuration R et, en position 5, dans la configuration S ; ou un énantiomère de celui-ci ; en présence d'une base aqueuse, ce qui mène à un sel de métal alcalin de formule 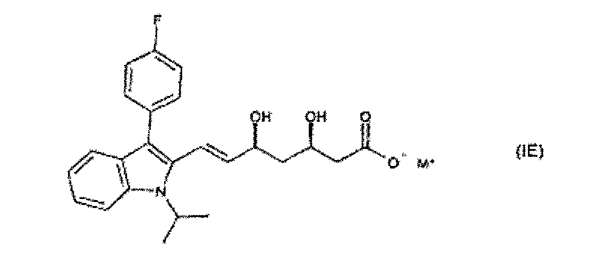 dans laquelle M représente le sodium, le lithium ou le potassium ; et
(2) traiter le sel de métal alcalin de formule IE avec un composé de calcium, ce qui mène au sel de calcium cristallin selon la revendication 7.

10. Procédé selon la revendication 9, dans lequel la base aqueuse à l'étape (1) est l'hydroxyde de sodium et M dans la formule IE représente le sodium, et dans lequel le composé de calcium à l'étape (2) est le chlorure de calcium.

11. Procédé de préparation d'un sel de calcium cristallin selon la revendication 7, lequel procédé comprend l'étape consistant à traiter un sel de métal alcalin de formule 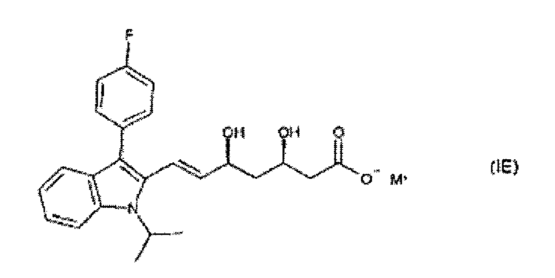 dans laquelle M représente le sodium, le lithium ou le potassium ; et le groupe hydroxyle est, en position 3, dans la configuration R et, en position 5, dans la configuration S ; ou un énantiomère de celui-ci ; ou un hydrate de celui-ci ; avec un composé de calcium, ce qui mène au sel de calcium cristallin selon la revendication 7.

12. Procédé selon la revendication 11, dans lequel M dans la formule IE représente le sodium et le composé de calcium est le chlorure de calcium.

13. Composition pharmaceutique comprenant une quantité efficace sur le plan thérapeutique d'un sel de calcium selon la revendication 7, en combinaison avec un ou plusieurs supports acceptables sur le plan pharmaceutique.

14. Utilisation d'un sel de calcium selon l'une quelconque des revendications 1 à 8, pour la fabrication d'un médicament destiné à la prévention et/ou au traitement de l'hypercholestérolémie, de l'hyperlipoprotéinémie, de la dyslipidémie et de l'athérosclérose.
